# EUROPÄISCHE PATENTSCHRIFT

(11) **EP 3 411 502 B1**
(45) Veröffentlichungstag und Bekanntmachung des Hinweises auf die Patenterteilung: **06.04.2022**
(21) Anmeldenummer: 17714635.4
(22) Anmeldetag: 01.02.2017
(51) Int. Cl.: C12Q 1/68, C12N 15/66, C12N 15/10

(54) **VERFAHREN, TRÄGER UND KIT ZUR EIN-TOPF-EIN-SCHRITT-ASSEMBLIERUNG VON DNA-MOLEKÜLEN**
METHOD, SUBSTRATE AND KIT FOR ONE-POT ONE-STEP ASSEMBLY OF DNA MOLECULES
PROCÉDÉ, SUBSTRAT ET KIT POUR L'ASSEMBLAGE DE TYPE « 1 TUBE-1 ÉTAPE » DE MOLÉCULES D'ADN

(30) Priorität: 04.02.2016 DE 102016101948
(43) Veröffentlichungstag der Anmeldung: 12.12.2018
(73) Patentinhaber: Leibniz-Institut für Pflanzenbiochemie (IPB), 06120 Halle (Saale) (DE)
(72) Erfinder: MARILLONNET, Sylvestre, 06126 Halle (DE)
(74) Vertreter: Stüven, Ralf
(86) Internationale Anmeldenummer: PCT/DE2017/200014
(87) Internationale Veröffentlichungsnummer: WO 2017/133738

(56) Entgegenhaltungen:
- WO-A1-90/03959
- WO-A1-2012/010708
- WO-A1-2014/004393
- WO-A1-2015/011203
- WO-A1-2015/057330
- ERNST WEBER ET AL: "A Modular Cloning System for Standardized Assembly of Multigene Constructs", PLOS ONE, Bd. 6, Nr. 2, 18. Februar 2011 (2011-02-18), Seite e16765, XP055110994, ISSN: 1932-6203, DOI: 10.1371/journal.pone.0016765 in der Anmeldung erwähnt
- ENGLER C ET AL: "A one pot, one step, precision cloning method with high throughput capability", PLOS ONE, PUBLIC LIBRARY OF SCIENCE, US, Bd. 3, Nr. 11, 5. November 2008 (2008-11-05), Seiten E3647-1, XP002613221, ISSN: 1932-6203, DOI: 10.1371/JOURNAL.PONE.0003647
- SIYING MA ET AL: "DNA synthesis, assembly and applications in synthetic biology", CURRENT OPINION IN CHEMICAL BIOLOGY, Bd. 16, Nr. 3-4, 1. August 2012 (2012-08-01), Seiten 260-267, XP055212288, ISSN: 1367-5931, DOI: 10.1016/j.cbpa.2012.05.001
- Nicola J Patron ET AL: "Standards for plant synthetic biology: a common syntax for exchange of DNA parts", New Phytologist, 1. Oktober 2015 (2015-10-01), Seiten 13-19, XP055372645, England DOI: 10.1111/nph.13532 Gefunden im Internet: URL:http://onlinelibrary.wiley.com/store/1 0.1111/nph.13532/asset/nph13532.pdf?v=1&t= j2pwv4kj&s=79c87eb366c615bda39b895b70e2643 95f9a2dd1

## Beschreibung

Die Erfindung betrifft ein Verfahren zur Ein-Topf-Ein-Schritt-Assemblierung von zwei oder mehr DNA-Molekülen zu mindestens einem rekombinanten DNA-Molekül sowie einen Träger und ein Kit hierfür.

Das Ziel der synthetischen Biologie ist ein Engineering von lebenden Organismen mit neuartigen Phänotypen, die in der Natur nicht existieren. Effiziente Methoden für das Zusammenfügen von DNA-Fragmenten, die für die Erstellung von Multigenkonstrukten erforderlich sind, stellen fundamentale Werkzeuge für die synthetische Biologie dar. Mehrere geeignete Methoden der Neukombination sind in den letzten Jahren entwickelt worden einschließlich der "Gibson Assembly"-Methode (Gibson et al. 2009, Nature Methods 6, 343-345) und des "Golden Gate Cloning" (Engler et al. 2008, Plos ONE 3 (11): e3647, doi:10.1371/journal.pone.0003647). Beide Methoden erlauben das Zusammenfügen von multiplen DNA-Fragmenten in einer Ein-Topf-Ein-Schritt-Reaktion mit einer extrem hohen Effizienz. Mit beiden Methoden enthalten die meisten Kolonien nach einer Transformation der Neukombinationsreaktion in kompetente E.-coli-Zellen das erwartete Konstrukt. Ein auf dem Golden-Gate-Cloning-Verfahren basierendes modulares und hierarchisches Klonierungssystem ist auch in Weber et al., 2011, PLoSONe 6(2): e16765 (doi: 10.1371/journal.pone.0016765) beschrieben.

Obwohl die Verfügbarkeit effizienter DNA-Neukombinations-Methoden das Zusammenfügen von DNA-Konstrukten erleichtern kann, ist die Gestaltung einer Klonierungsstrategie immer noch ein limitierender Faktor. Große Multigenkonstrukte erfordern die Planung aufeinanderfolgender Klonierungsschritte um Schritt für Schritt größere Konstrukte mit mehr Genen zu generieren. Irgendwann enthalten große Konstrukte mit hoher Wahrscheinlichkeit multiple Schnittstellen für eine Vielzahl von Restriktionsenzymen, was deren Verwendung für das Einfügen weiterer Gene in das Konstrukt ausschließen kann und die Planung einer Klonierungsstrategie zunehmend schwierig macht. Die Anwendung von Standardisierungprinzipien in solchen Systemen kann eine Lösung dieses Problems ermöglichen. Standardisierung von Teilen besteht in der Definition von Standardstrukturen für Konstrukte, die die Sequenzen für grundlegende genetische Elemente wie Promotoren, codierende Sequenzen und Terminatoren enthalten. Solche biologischen Standardteile (Grundbausteine) werden von Restriktionsschnittstellen flankiert. Restriktionsenzyme für diese Stellen werden dann für das Zusammenfügen von diesen DNA-Fragmenten verwendet, um z.B. Transkriptionseinheiten zu generieren. Einige Restriktionsschnittstellen können von internen Sequenzen der Basismodule auch bewusst eliminiert werden, um die Verwendung der betreffenden Enzyme für weitere Assemblierungsschritte zu ermöglichen. Mehrere Standardprozeduren sind vorgeschlagen worden, die sich in der Wahl der die Basismodule flankierenden Restriktionsschnittstellen unterscheiden (Cassini et al. 2015, Nat Rev Mol Cell Biol 16(9) 568-576). Das "Modular Cloning (MoClo)"-System ist ein Beispiel für eine solche standardisierte Klonierungsprozedur (Weber et al. 2011, Plos ONE, 6(2): e16765, doi: 10.1371/journal.pone.0016765; Patron et al. 2015, New Phytol., 208, 13-19).

Die Verwendung von standardisierten Einzelteilen erleichtert das Zusammenfügen großer Konstrukte, da eine universelle Klonierungsstrategie eingeschlagen werden kann, die unabhängig von der Natur der einzelnen Teile ist, die neu kombiniert werden sollen. Sie erleichtert ebenfalls die Wiederverwendung von Einzelteilen in vielen unterschiedlichen Konstrukten, da alle Module vom gleichen Typ die gleichen Assemblierungseigenschaften haben und daher gegen andere Teile vom gleichen Typ ausgetauscht werden können. Beispielsweise kann ein Promotor, der als Standardteil kloniert ist, für die Assemblierung von Transkriptionseinheiten gegen jeden anderen Standardpromotor ausgetauscht werden und es kann die gleiche Assemblierungprozedur verwendet werden.

Ein zusätzlicher Vorteil solcher standardisierter Module ist die Tatsache, dass diese Einzelteile auch von Wissenschaftlern anderer Laboratorien verwendet werden können, die die gleichen Standards benutzen. Man kann somit erwarten, dass die Anzahl solcher Module erheblich wächst, da Teile mit den gleichen Standards von vielen verschiedenen Laboratorien konstruiert und benutzt werden können. Die Anzahl solcher biologischer Module, die für Zwecke der synthetischen Biologie generiert werden können ist theoretisch unbegrenzt. Beispielsweise könnten alle codierenden und regulatorischen Sequenzen irgendeines lebenden Organismus kloniert werden und als standardisierte biologische Module genutzt werden solange sie mit einem definierten Standard kloniert werden. Zusätzlich zu Modulen abgeleitet von lebenden Organismen können auch synthetische Module, die in der Natur nicht existieren, generiert werden. Beispielsweise können Bibliotheken von synthetischen Promotoren erstellt werden, die Segmente definierter Sequenzen sowie auch Segmente degenerierter Sequenzen enthalten (Brückner et al. 2015, Plant J. 82, 707-716).

Sobald solche biologischen Grundmodule erstellt worden sind, müssen sie für den späteren Gebrauch gesichert werden. Solche DNA-Module werden gewöhnlich als gereinigte Plasmid-DNA in einer Pufferlösung gefroren bei -20 °C aufbewahrt. Alternativ können diese Module auch als Glycerinkulturen bei -80 °C aufbewahrt werden. Die Glycerinkultur enthält einen Bakterienstamm, der mit der das Standardmodul enthaltenden Plasmid-DNA transformiert worden ist. In diesem Fall muss vor der Verwendung in einer neuen Klonierung DNA aus frisch kultivierten E.-coli-Zellen extrahiert werden. Da üblicherweise nicht die gesamte extrahierte und gereinigte Plasmid-DNA in einem Experiment verwendet wird, wird der Rest gewöhnlich bei -20 °C wie oben beschrieben für den weiteren Gebrauch eingefroren.

Es gibt zwei Probleme bei der Aufbewahrung von gereinigter Plasmid-DNA bei -20 °C. Erstens, die Aufbewahrung von DNA bei niedriger Temperatur, d.h. -20°C, ist sehr kostenaufwendig. Während das für eine begrenzte Anzahl von Proben noch akzeptabel ist, würde dieses für eine sehr große Probenzahl sehr teuer werden und eine große Anzahl von Gefriergeräten erfordern. Ein zweites Problem ist die Verschlechterung der DNA-Qualität mit steigender Lagerdauer selbst bei einer Lagerung bei -20°C. Dieses kann zu reduzierter Klonierungseffizienz oder sogar zum Scheitern von DNA-Assemblierungsreaktionen führen.

Eine Alternative zur Lagerung von DNA oder Bakterienstämmen bei -20°C ist deren Aufbewahrung in trockener Form bei Raumtemperatur. Für Klonierungszwecke muss diese DNA bisher allerdings zunächst in Wasser oder Puffer solubilisiert und dann in E. coli transformiert werden. Eine bakterielle Kolonie mit dem transformierten Plasmid wird dann in Flüssigmedium kultiviert, Plasmid-DNA extrahiert und schließlich die DNA-Konzentration der Plasmidpräparation gemessen. Nachfolgend kann die DNA für Klonierungszwecke verwendet werden. Diese Prozesse sind sehr zeitaufwendig und arbeitsintensiv.

In der WO 2012/010708 A1 ist offenbart, Komponenten für molekularbiologische Reaktionen, beispielsweise PCR-basierte Amplifizierungsreaktionen, vor dem Reaktionsstart auf einem festen Träger, vorzugsweise einer porösen Filterscheibe aus Polyethylen, in räumlich getrennten Kompartimenten trocken zu lagern.

Die WO 2015/057330 A1 beschreibt die direkte Quantifizierung von Nukleinsäuren für forensische Zwecke in Papier- oder Filterpapier-Proben mittels PCR-Amplifikation ohne vorherige Extraktion.

WO 2014/004393 A1 offenbart die Synthese von Polynukleotiden aus synthetischen Oligonukleotiden. Zur Synthese der Oligonukleotide können diese auf porösen Trägern, z.B. Filterpapier, immobilisiert sein.

Es besteht nach wie vor ein Bedarf, Assemblierungs- bzw. Klonierungsverfahren zu verbessern, insbesondere zu vereinfachen und kostengünstiger zu machen. Aufgabe der vorliegenden

Erfindung ist es daher, ein einfaches und kostengünstiges Assemblierungsverfahren für DNA-Moleküle bereit zu stellen.

Zur Lösung der Aufgabe wird erfindungsgemäß in einem ersten Aspekt ein Verfahren zur Ein-Topf-Ein-Schritt-Assemblierung von zwei oder mehr DNA-Molekülen zu mindestens einem rekombinanten DNA-Molekül bereit gestellt, wobei die zwei oder mehr zu assemblierenden DNA-Moleküle jeweils flankiert sind von Restriktionsendonuklease-Typ-IIs-Schnittstellen mit entgegengesetzter Orientierung und in trockener Form auf oder in mindestens einem Träger vorliegend in einem Reaktionsgefäß mit einem geeigneten Reaktionsmedium zusammen gebracht werden, und wobei der Träger so ausgestaltet und dimensioniert ist, dass er individuell als Ganzes und unverändert in einem Mikroreaktionsgefäß angeordnet werden kann und ein Filterpapierstück oder ein Zellulosepartikel ist oder umfasst.

Das erfindungsgemäße Verfahren schlägt die Verwendung von kostengünstig lagerfähigen trockenen DNA-Aliquots zum Einmalgebrauch für die direkte DNA-Assemblierung vor. Die Erfindung nutzt die Stabilität und die niedrigen Kosten einer DNA-Lagerung in trockenem Zustand bei Raumtemperatur aus, und erfordert keine Transformation bakterieller Zellen und die Extraktion von Plasmid-DNA vor der Klonierung.

Das erfindungsgemäße Verfahren sieht die Nutzung von DNA vor, die im trockenen Zustand auf oder in einem geeigneten Träger vorliegt, vorzugweise in Aliquots in einer definierten Menge. Besonders bevorzugt ist, wenn jedes Aliquot einer trockenen DNA-Probe für eine Assemblierungs- bzw. Klonierungsreaktion ausreichend ist. Diese definierte DNA-Menge ist z.B. für eine leichte Handhabung auf einem kleinen Stück Filterpapier oder einem Zellulosepartikel, beispielsweise einem Partikel aus mikrokristalliner Zellulose, aufgebracht. Das Klonieren würde dann beispielsweise durch Zugabe eines ersten Filterpapierstückchens oder Zellulosepartikels, beispielsweise eines Partikels aus mikrokristalliner Zellulose, mit beispielsweise einem Insert (codierendes Fragment) in ein Reaktionsgefäß erfolgen, gefolgt von der Zugabe eines zweiten Filterpapiers oder Zellulosepartikels mit einem geeigneten Vektor. Für den Fall, dass das Klonieren mit dem "Golden-Gate-System" mit Restriktionsenzym und Ligase durchgeführt wird, würde die Zugabe von Restriktionsligase-Puffer, Restriktionsenzym und Ligase folgen. Bei Verwendung der "Gibson Assembly"-Methode könnte die Zugabe von Exonuklease, DNA-Polymerase und DNA-Ligase folgen. Selbstverständlich kann auch das Reaktionsmedium in einem Reaktionsgefäß vorgelegt werden und die DNA-Aliquots werden anschließend hinzugefügt. Wenn alle Komponenten gemeinsam im Reaktionsgefäß vorliegen, kehrt die trockene DNA in eine lösliche Form zurück und kann sofort geschnitten und ligiert werden, was in der Assemblierung des gewünschten Produkts resultiert.

Unter einem "Verfahren zur Ein-Topf-Ein-Schritt-Assemblierung von zwei oder mehr DNA-Molekülen" oder einer "Ein-Topf-Ein-Schritt-Assemblierungsmethode" werden hier Verfahren wie das "Golden Gate"- oder "Gibson-Assembly"-Verfahren verstanden, bei denen die Synthese des resultierenden rekombinanten DNA-Moleküls, beispielsweise eines Klonierungsvektors, in einem Schritt und in einem Reaktionsgefäß durchgeführt werden kann. Solche Verfahren unterscheiden sich von anderen Standard-Klonierungsmethoden dadurch, dass DNA-spaltende bzw. -abbauende und DNA-ligierende bzw. aufbauende Prozesse nebeneinander ablaufen können, ohne dass die Synthese dadurch beeinträchtigt würde. Beispielsweise erfordert das "Golden-Gate"-Verfahren keine getrennten Schritte für Restriktionsverdau und Ligation, während dies bei anderen Standard-Klonierungsmethoden nötig ist, was darauf zurückgeht, dass ligierte DNA-Fragemente nach der Ligation neu verdaut werden können, da die Restriktionsenzym-Schnittstellen für die Klonierung durch den Ligationsschritt wiederhergestellt werden.

Der Begriff "Assemblierung von zwei oder mehr DNA-Molekülen" bedeutet die In-vitro-Synthese von zwei, drei, vier oder auch mehr, vorzugsweise unterschiedlichen, DNA-Molekülen zu einem einzigen DNA-Molekül. Beispielsweise kann ein Empfängervektor mit DNA-Molekülen, die codierende oder regulatorische DNA-Sequenzen bilden oder enthalten, zu einem Klonierungsvektor assembliert werden. Der Begriff "zwei oder mehr DNA-Moleküle" ist dabei selbstverständlich nicht einschränkend so zu verstehen, dass er sich nur auf die genannte Anzahl von Molekülen bezieht, beispielsweise ein einzelnes DNA-Molekül A und ein einzelnes DNA-Molekül B. Vielmehr ist die Formulierung so zu verstehen, dass mindestens ein erstes DNA-Molekül (z.B. ein Empfängervektor) mit mindestens einem zweiten, vorzugsweise vom ersten verschiedenen, DNA-Molekül (z.B. mit einer codierenden DNA-Sequenz) assembliert wird. Ein mit einem anderen DNA-Molekül zu assemblierendes DNA-Molekül kann beispielsweise auch in einem größeren Nukleinsäuremolekül bzw. -konstrukt, das linear oder ringförmig geschlossen sein kann, z.B. in einem Plasmid, enthalten sein, aus dem es im Verlauf der Reaktion herausgeschnitten und mit dem anderen DNA-Molekül assembliert wird.

Unter einem "geeigneten Reaktionsmedium" wird hier ein vorzugsweise wässriges flüssiges Medium verstanden, in dem die trockenen DNA-Moleküle sich lösen und unter geeigneten Bedingungen miteinander reagieren können. Es kann sich beispielsweise um eine Pufferlösung handeln, die ggfs. geeignete Enzyme enthält wie z.B. DNA-Ligase und ein Restriktionsenzym des Typs IIs.

Unter einem "Mikroreaktionsgefäß" wird hier ein Reaktionsgefäß verstanden, das Probenvolumina im Bereich von Nanolitern, Mikrolitern und maximal wenigen Millilitern fasst, beispielsweise von 0,005 bis 2 ml, vorzugsweise von beispielsweise 0,01 ml bis 1,5 ml oder von 0,05 bis 0,5 ml. Es kann sich beispielsweise um Gefäße aus Kunststoff, z.B. Polypropylen (PP), mit oder ohne Deckel handeln. Der Begriff umfasst aber auch die Näpfe ("wells") von beispielsweise Mikrotiterplatten oder dergleichen.

Wenn hier von einem "DNA-Molekül in trockener Form" gesprochen wird, so bedeutet dies, dass der Träger mit dem darauf oder darin befindlichen DNA-Molekül vorzugsweise einen möglichst geringen Wassergehalt aufweist. Im Falle beispielsweise eines einfachen Filterpapiers als Träger kann das bedeuten, dass der Wassergehalt im Wesentlichen dem entspricht, der sich bei einfacher Lagerung bei Raumtemperatur und Umgebungsfeuchtigkeit einstellt. Im Falle beispielsweise eines beschichteten Trägers kann der Wassergehalt in der Umgebung der DNA-Moleküle aber auch deutlich unterhalb der Umgebungsfeuchte liegen. Es ist auch möglich, auf, in oder an dem Träger oder in dessen Umgebung ein geeignetes Trocknungsmittel vorzusehen, um das DNA-Molekül trocken zu halten.

Mit dem erfindungsgemäßen Verfahren kann auch die Klonierung von mehr als einem Insert in einen Vektor unter Einsatz trockener DNA-Aliquots in einem Schritt durchgeführt werden. Beispielsweise kann die Assemblierung einer Transkriptionseinheit mit einem Promotor, einer codierenden Sequenz und einem Terminator in einem ausgewählten Vektor mittels vier trockener DNA-Proben durchgeführt werden, eine für den Empfängervektor sowie drei für jede der Grundeinheiten der Transkriptionseinheit. Alle vier Fragmente, die jeweils auf getrennten Trägern, z.B. Filterpapierstückchen oder Zellulosepartikeln, oder auch zumindest teilweise gemeinsam auf jeweils einem Träger, z.B. Filterpapierstück oder Zellulosepartikel, vorliegen können, würden zusammen mit Puffer und Enzymen in ein Reaktionsgefäß gegeben werden, analog der Subklonierung eines Fragments wie oben beschrieben. Es ist zwar insbesondere zur Herstellung eines bestimmten rekombinanten DNA-Moleküls bevorzugt, dass jedes der zu assemblierenden DNA-Moleküle auf/in einem separaten Träger, vorzugsweise in einer für die Klonierung ausreichenden Menge, in getrockneter Form vorliegt. Grundsätzlich ist es aber auch möglich, dass alle oder ein Teil der DNA-Moleküle auf/in einem gemeinsamen Träger vorliegen, beispielsweise der Empfängervektor auf/in einem ersten Träger und regulatorische/codierende DNA-Moleküle auf/in einem gemeinsamen zweiten Träger. Es ist auch möglich, auf/in einem Träger nur einen Typ DNA-Molekül vorzusehen, um eine Bibliothek solcher DNA-Moleküle auf/in einem, oder gegebenenfalls auch mehreren Trägern, bereit stellen zu können. Beispielsweise könnte auf/in einem oder gegebenenfalls mehreren Trägern eine Anzahl von mehreren, z.B. 10, 20, 50 oder 100, verschiedenen Promotoren angeordnet sein. Dies kann vorteilhaft sein, um beispielsweise eine entsprechende Bibliothek unterschiedlicher DNA-Konstrukte herzustellen, die sich im Promotor unterscheiden. Ebenso ist es möglich, zwei oder mehr solcher Bibliotheken unterschiedlicher Typen von DNA-Molekülen bereit zu stellen, um auf einfache Weise eine Vielzahl von verschiedenen Konstrukten herstellen zu können.

Das Verfahren eignet sich in besonderer Weise zur Durchführung von Assemblierungen von standardisierten DNA-Molekülen. Dabei handelt es sich um DNA-Moleküle, die in einer standardisierten Form vorliegen, in der sie beispielsweise direkt in einem "Golden-Gate"- oder "Gibson-Assembly"-Verfahren einsetzbar sind. Insbesondere sind bei solchen standardisierten DNA-Molekülen die Verbindungsstellen, d.h. die zu ligierenden Enden, so aufeinander abgestimmt, dass eine Assemblierung auch mehrerer DNA-Moleküle in einem Ein-Topf-Ein-Schritt-Verfahren, beispielsweise einem "Golden-Gate"- oder "Gibson-Assembly"-Verfahren ermöglicht ist.

Besonders bevorzugt handelt es sich um DNA-Moleküle, die für das "Golden-Gate"-Verfahren geeignet und vorzugsweise dafür standardisiert sind. Dabei sind die auf oder in dem Träger vorliegenden zwei oder mehr DNA-Moleküle vorzugsweise jeweils flankiert von Restriktionsendonuklease-Typ-IIs-Schnittstellen mit entgegengesetzter Orientierung. Restriktionsendonukleasen vom Typ-IIs sind Restriktionsendonukleasen, deren Schnittstelle zu einer Seite außerhalb von ihrer asymmetrischen nicht-palindronischen Erkennungssequenz liegt. Typ-IIs-Restriktionsendonukleasen sind dem Fachmann bekannt. Beispiele für Restriktionsendonukleasen von Typ II umfassen BsaI, BpiI, BsmBI, SapI und FokI. Der Begriff "Orientierung" bezieht sich hier auf die Richtung von der Erkennungssequenz der Restriktionsendonuklease auf der DNA hin zur außerhalb davon zu einer Seite liegenden Schnittstelle.

Wie bereits angegeben, können die DNA-Moleküle auf/in dem Träger in einem größeren DNA-Konstrukt (linear oder kreisförmig) vorliegen. Die Schnittstellen der Restriktionsendonuklease vom Typ IIs sind in diesen Konstrukten vorzugsweise so orientiert, dass nach dem Schneiden das DNA-Molekül ohne die flankierenden Erkennungssequenzen resultiert. Im Falle eines Emfängervektors sind die Schnittstellen beispielsweise so orientiert, dass bei der durch die Restriktionsendonuklease katalysierten Spaltungsreaktion aus dem Konstrukt ein Empfängervektor hervorgeht, der keine Erkennungssequenz der Restriktionsendonuklease vom Typ-IIs mehr aufweist. Die Erkennungssequenzen der Restriktionsendonuklease vom Typ IIs liegen daher im Falle eines Empfängervektors als einem der zu assemblierenden DNA-Moleküle außerhalb des Emfängervektors, während die Schnittstellen zum Emfängervektor gerichtet sind. Bei Nicht-Vektor-DNA, beispielsweise regulatorischen oder codierenden Sequenzen, z.B. Promotoren, Terminatoren etc., verhält es sich entsprechend so, dass die Schnittstellen vorzusweise zu dem DNA-Molekül gerichtet sind. Derartige DNA-Moleküle sind besonders gut geeignet für das "Golden-Gate"-Verfahren.

Als Träger für die DNA kommen beispielsweise Filterpapier oder Zellulosepartikel (Zellulosekügelchen bzw. Zellulose-Beads) in Frage, z.B. Kügelchen (Beads) aus mikrokristalliner Zellulose (MCC). Es ist lediglich erforderlich, den Träger mit einer definierten DNA-Menge zu beladen und den beladenen Träger handhaben zu können. Es ist auch möglich, beispielsweise Zellulosekügelchen mit der DNA zu beladen und die Zellulosekügelchen zu beschichten, beispielsweise mit Trehalose und/oder anderen Hilfsmitteln, wie z.B. Polyvinylalkohol. Geeignete Formulierungen für Beschichtungen sind dem Fachmann bekannt. Vorzugsweise wird die Formulierung oder Beschichtung so gewählt, dass sie sich in dem Reaktionsmedium leicht auflöst, um das DNA-Molekül freizusetzen. Der Träger kann eine beliebige Größe aufweisen, ist aber so ausgestaltet und dimensioniert ist, dass er individuell als Ganzes und unverändert in einem Mikroreaktionsgefäß angeordnet werden kann, Der Träger kann ggfs. beispielsweise auch farblich markiert sein, um eine einfache optische Unterscheidung zu ermöglichen.

In einem zweiten Aspekt betrifft die Erfindung auch einen Träger für den Einsatz in einem erfindungsgemäßen Verfahren nach dem ersten Aspekt, umfassend mindestens ein in trockener Form vorliegendes DNA-Molekül, das mit einem anderen DNA-Molekül assemblierbar ist und jeweils flankiert ist von Restriktionsendonuklease-Typ-IIs-Schnittstellen mit entgegengesetzter Orientierung, wobei der Träger mit dem DNA-Molekül so ausgestaltet und dimensioniert ist, dass er individuell als Ganzes und unverändert in einem Mikroreaktionsgefäß angeordnet werden kann, und wobei der Träger ein Filterpapierstück oder ein Zellulosepartikel mit dem darin enthaltenen DNA-Molekül ist oder umfasst. Beispiele für einen möglichen Träger sind ein Filterpapierstück oder ein Zellulosepartikel geeigneter Form und Größe. Es ist auch möglich, eine Mischung aus dem DNA-Molekül und beispielsweise einem Zucker, z.B. Trehalose, oder einer anderen Substanz oder Zusammensetzung herzustellen, die bei Trocknung eine das DNA-Molekül umfassende Matrix bildet, die Mischung auf einen Träger, z.B. Filterpapier oder Zellulosekügelchen, aufzubringen und zu trocknen. Es ist zwar bevorzugt, dass auf einem Träger lediglich ein bestimmtes DNA-Molekül (z.B. einen Promotor, einen Terminator oder dergleichen, ggfs. in einem DNA-Konstrukt, z.B. einem Plasmid enthalten) vorliegt. Es ist grundsätzlich aber auch möglich, zwei oder mehrere DNA-Moleküle auf einem Träger vorzusehen. Vorzugsweise umfasst der Träger das DNA-Molekül in einer Menge, die für eine Assemblierungsreaktion insbesondere nach dem "Golden-Gate"-Verfahren ausreicht.

Die Formulierung, wonach der Träger "so ausgestaltet und dimensioniert ist, dass er individuell als Ganzes und unverändert in einem Mikroreaktionsgefäß angeordnet werden kann" bedeutet insbesondere, dass der Träger mit dem darauf oder darin befindlichen DNA-Molekül ausreichend klein und stabil ist, um per Hand oder auch maschinell, z.B. per Roboter, in ein Mikroreaktionsgefäß, z.B. ein 1,5-ml-Reaktionsgefäß, verbracht zu werden. Beispiele für geeignete Träger sind quadratische Filterpapierstücke mit beispielsweise 0,5x0,5 mm, 1×1 mm, 1,5x1,5 mm oder 2x2 mm Kantenlänge, oder runde Filterpapierstücke oder Zellulosebeads mit beispielsweise einem Durchmesser von 0,5-3, vorzugsweise 0,5-2,5, besonders bevorzugt 0,5-2 mm Durchmesser. Es kommen aber grundsätzlich auch sehr viel kleinere Träger, beispielsweise von unter 0,5 mm Kantenlänge oder Durchmesser in Frage.

Die Formulierung, wonach der Träger "das DNA-Molekül in einer Menge umfasst, die für eine Assemblierungsreaktion ausreicht" bedeutet insbesondere, dass auf oder (beispielsweise im Fall einer Tablette) in dem Träger in trockener Form eine solche Menge des jeweiligen DNA-Moleküls vorhanden ist, dass nach Lösung des DNA-Moleküls in der Reaktionslösung eine Assemblierungsreaktion stattfindet. Eine ausreichende Menge ist insbesondere eine solche Menge, die ausreicht, um eine Menge rekombinantes DNA-Molekül zu erzeugen, die für beispielsweise eine Transformation von E-coli-Zellen ausreicht. Eine ausreichende Menge kann beispielsweise eine auf/in dem Träger vorliegende Menge von 1-100 oder mehr fmol sein, beispielsweise eine Menge von 5, 10, 20, 25, 30 oder 40 fmol.

Die Erfindung stellt in einem dritten Anspekt auch ein Kit zur Durchführung des erfindungsgemäßen Verfahrens bereit, wobei das Kit mindestens einen Träger gemäß dem oben beschriebenen zweiten Aspekt der Erfindung umfasst. Vorzugsweise umfasst das Kit zwei oder mehr zu assemblierende DNA-Moleküle, wobei jedes der zwei oder mehr DNA-Moleküle vorzugsweise auf/in einem separaten Träger in einer für die Assemblierung ausreichenden Menge vorliegt.

Bei den DNA-Molekülen handelt es sich vorzugsweise um standardisierte DNA-Moleküle, besonders bevorzugt um an das "Golden-Gate"-Verfahren angepasste DNA-Moleküle, wie oben beschrieben. Das erfindungsgemäße Kit kann, muss aber nicht, alle für eine bestimmte beabsichtigte Assemblierung erforderlichen DNA-Moleküle umfassen. Beispielsweise kann das Kit auch einen Satz von Promotoren enthalten, jedoch keinen Terminator und/oder einen Vektor. Das Kit kann auch bloß einen Satz eines Typs von DNA-Molekülen umfassen, beispielsweise einen Satz von Promotoren, wie oben bereits erwähnt.

Bevorzugt umfasst das erfindungsgemäße Kit auch a) eine Restriktionsendonuklease vom Typ IIs und eine DNA-Ligase oder b) eine Exonuklease, eine DNA-Polymerase und eine DNA-Ligase. In diesen bevorzugten Ausführungsformen ist das Kit besonders geeignet zur Durchführung des "Golden-Gate"- oder "Gibson-Assembly"-Verfahrens für die Klonierung. Die Erfindung wird im Folgenden anhand der beigefügten Figuren und Ausführungsbeispielen rein zu Veranschaulichungszwecken näher beschrieben.

Figur 1 Schematische Darstellung einer Ausführungsform des erfindungsgemäßen Verfahrens.

Figur 2 Schematischer Aufbau von Insert- und Vektor-Plasmiden für den Einsatz bei der in Figur 1 dargestellten Ausführungsform des erfindungsgemäßen Verfahrens für den Fall einer "Golden-Gate"-Klonierung.

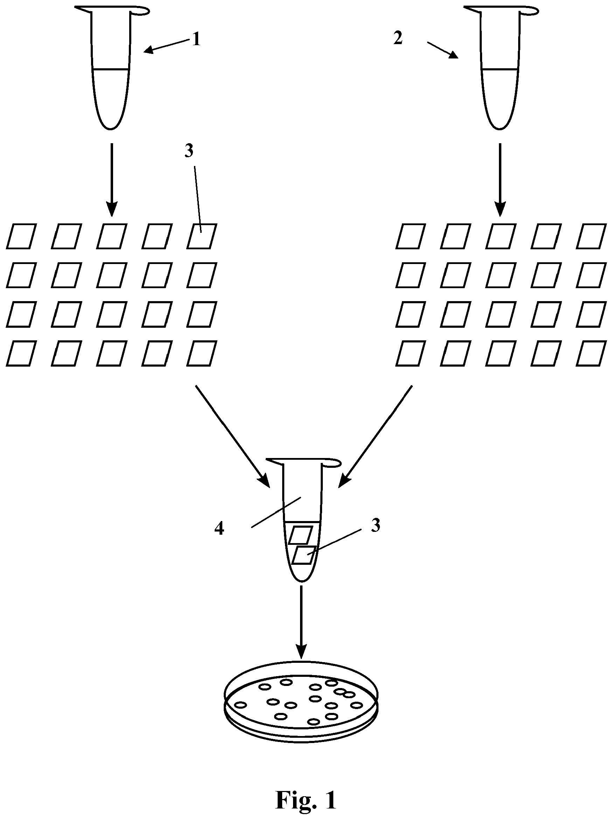

Figur 1 zeigt schematisch eine Ausführungsform des erfindungsgemäßen Verfahrens. Aliquots einer DNA-Präparation 1 des Empfängervektors und einer DNA-Präparation 2 eines Inserts werden in geeigneter Menge auf separate Filterpapierscheiben 3 aufgebracht. Die mit DNA beladenen Filterpapierscheiben 3 werden getrocknet und bis zur Verwendung in trockener Umgebung z.B. bei Raumtemperatur gelagert. Jeweils eine Filterpapierscheibe 3 mit Vektor- und Insertpräparation wird in ein Reaktionsgefäß 4 gegeben und mit einer Reaktionslösung in Kontakt gebracht, die beispielsweise Restriktionsenzym und Ligase enthält. Nach Inkubation der Assemblierungsreaktionsmischung wird beispielsweise E. coli mit dem in dem flüssigen Überstand enthaltenen rekombinanten Plasmid transformiert.

### Ausführungsbeispiele

### Beispiel 1

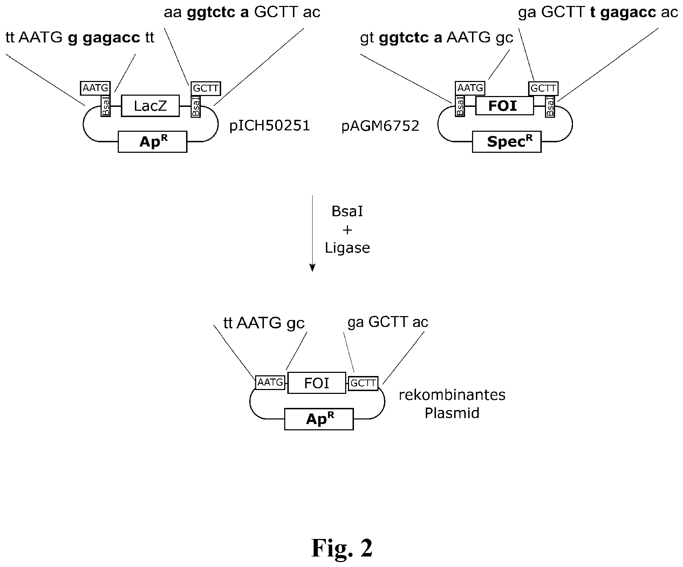

40 fmol (2,4 µL) von Plasmid pICH50251 (Aufbau siehe Fig. 2 links; dargestellte Sequenzabschnitte in den SEQ ID NO: 1, 2) wurden auf ein kleines Stückchen Filterpapier 3 (quadratisch mit 2 mm Kantenlänge) pipettiert, das aus einem größeren Filterpapier (Sartorius, Klasse 6, 80g/m2 FT 3-312-070) ausgeschnitten wurde. 40 fmol (1,8 µL) Plasmid pAGM6752 (s. Figur 2, rechts; dargestellte Sequenzabschnitte in den SEQ ID NO: 3, 4) wurden auf ein zweites Filterpapierstückchen 3 von ähnlicher Größe und Beschaffenheit pipettiert. Die beiden Filterpapierstückchen 3 wurden dann bei Raumtemperatur getrocknet und für 24 h trocken aufbewahrt.

Die beiden Filterpapierstückchen 3 wurden in ein 1,5 mL Reaktionsgefäß 4 gegeben und mit 3 µL 10× Promega Ligasepuffer, 1,5 µL *Bsa*I (15 Units, NEB), 1,5 µL Ligase (4,5 Units, Promega) und 24 µL Wasser für ein Gesamtreaktionsvolumen von 30 µL versetzt. Das Reaktionsgefäß 4 wurde 1 h bei 37°C inkubiert, gefolgt von 5 min bei 50 °C und 5 min bei 80 °C.

Die gesamte Ligationsreaktion wurde in 50 µL E. coli DH10b kompetente Zellen mittels Hitzeschock transformiert. 500 µL LB Flüssigmedium wurden zu den Zellen zugefügt und für 45 min bei 37°C inkubiert. 50 µL dieses Transformationsansatzes wurden auf Selektionsmedium ausplattiert. Eine ungefähr gleiche Anzahl von blauen und weißen Kolonien wurde erhalten (jeweils etwa 130 Kolonien). Vier weiße Kolonien wurden gepickt und in Flüssigmedium kultiviert. Daraus wurde Plasmid-DNA isoliert, mit Restriktionsverdau analysiert, und als korrespondierend zu dem erwarteten Konstrukt befunden.

Dieses Ergebnis zeigt, dass die Klonierung direkt aus der getrockneten, auf einen Filterpapierträger aufgebrachten DNA-Probe erfolgen kann, ohne die Notwendigkeit, den Vektor und das Insert-DNA-Fragment in Bakterienstämme für Plasmidpräparationen rückzutransformieren.

### Beispiel 2

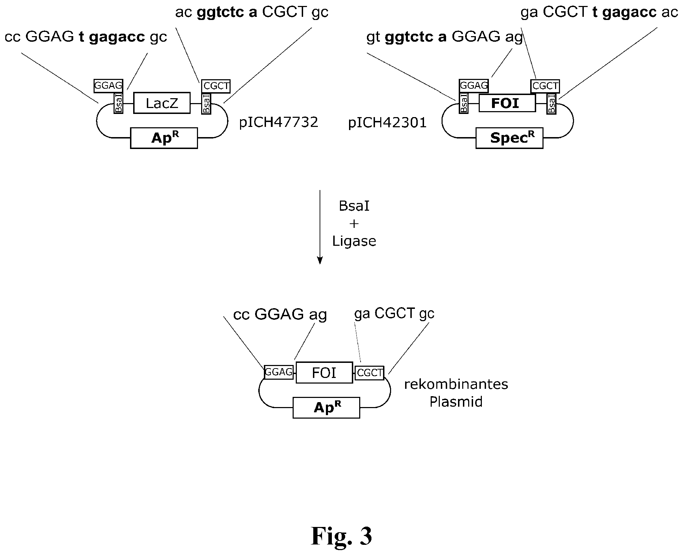

DNA von den Plasmiden pICH47732 (Insert; äquivalent zu Plasmid pICH50251 hinsichtlich der Restriktionsstellen für die "Golden Gate"-Assemblierung; Aufbau s. Fig. 3 links; dargestellte Sequenzabschnitte in den SEQ ID NO: 5, 6) und pICH42301 (Vektor; äquivalent zu dem Plasmid pAGM6752 hinsichtlich der Restriktionsstellen für die "Golden-Gate"-Assemblierung; s. Fig. 3 rechts; dargestellte Sequenzabschnitte in den SEQ ID NO: 7, 8) wurden unter Verwendung des Macherey-Nagel-Miniprep-Kits "NucleoSpin^{®} Plasmid" aufbereitet. Die DNA-Konzentrationen, die unter Verwendung eines "NanoDrop"-UV-Vis-Spektralphotometers (Thermo Fisher Scientific Inc.) gemessen wurden, betrugen 275 ng und 172 ng pro Mikroliter bzw. 89 und 82 fmol pro Mikroliter.

DNA für beide Plasmide wurde getrennt zu sterilen MCC-Beads, d.h. Kügelchen aus mikrokristalliner Zellulose (Cellets^{®} 700, HARKE Pharma GmbH), in zwei separaten Reaktionsgefäßen gegeben und jeweils vier gesonderten Behandlungen unterzogen:
Behandlung 1: Zu etwa 55 Kügelchen in einem 1,5-ml-Reaktionsgefäß wurden 10 µL DNA und 10 µL Wasser gegeben.
Behandlung 2: Zu etwa 55 Kügelchen in einem 1,5-ml-Reaktionsgefäß wurden 10 µL DNA und 10 µL einer 5% Trehalose-Lösung (steril) gegeben.
Behandlung 3: Zu etwa 55 Kügelchen in einem 1,5-ml-Reaktionsgefäß wurden 10 µL DNA und 10 µL einer 4% PVA-Lösung (4% Polyvinylalkohol in 200 mM Tris-HCl, pH 8,0; steril) gegeben.
Behandlung 4: Zu etwa 55 Kügelchen in einem 1,5-ml-Reaktionsgefäß wurden 10 µL DNA und 10 µL einer Trehalose-PVA-Lösung (2% Trehalose, 4% Polyvinylalkohol, in 200 mM Tris-HCl, pH 8,0; steril) gegeben.

DNA und Kügelchen der 8 Reaktionsgefäße wurden über Nacht bei Raumtemperatur luftgetrocknet. Die zugegebene DNA-Menge sollte für jedes Plasmid 15 bis 16 fmol pro Kügelchen betragen.

Am folgenden Tag wurden jeweils ein mit pICH47732 beschichtetes Kügelchen und ein mit pICH42301 beschichtetes Kügelchen (beide aus der gleichen Behandlung) in ein gemeinsames PCR-Reaktionsgefäß gegeben. Dazu wurden 12 µl steriles H₂O, 1,5 µL 10× Ligationspuffer, 1 µL Ligase und 0,5 µl BsaI-Restriktionsenzym gegeben. Das Reaktionsgefäß wurde verschlossen, mit den Fingern angestoßen und dann in einen Thermocycler mit den folgenden Parametern gebracht: 4 Stunden Inkubation bei 37 °C, gefolgt von 5 min Inkubation bei 50 °C und 5 min bei 80 °C. Der Überstand wurde mittels Hitzeschock in kompetente E.-coli-Zellen transformiert. 30 µL der 565 µL Transformationsmischung wurden auf LB-Platten plattiert, die X-Gal und Carbenicillin enthielten. Die Platten 1 bis 4 wiesen die folgenden Kolonien auf:
Behandlung 1: 155 weiße Kolonien, geschätzt 23250 für die gesamte Transformation.
Behandlung 2: 282 weiße Kolonien, geschätzt 42300 für die gesamte Transformation.
Behandlung 3: 196 weiße Kolonien, geschätzt 29400 für die gesamte Transformation.
Behandlung 4: 143 weiße Kolonien, geschätzt 21450 für die gesamte Transformation.

Für alle Behandlungen war die Mehrzahl der Kolonien weiß und eine Minderheit blau. Aus zwei weißen Kolonien pro Behandlung wurde DNA extrahiert. Bei allen wurde gefunden, dass sie das korrekte Konstrukt enthielten.

Das Klonieren unter Verwendung von MCC-Beads, die mit trockener DNA beschichtet sind, erwies sich somit als sehr effizient.

Weitere Experimente wurden durchgeführt, um eine längere Trocknungszeit vor dem Klonieren zu testen und die Zusammensetzung der DNA-Lösung zu variieren.

DNA für beide Plasmide wurde getrennt zu sterilen MCC-Beads (Cellets^{®} 700, HARKE Pharma GmbH) in zwei getrennten Reaktionsgefäßen gegeben und den folgenden drei Behandlungen unterzogen:
Behandlung 5: Zu etwa 55 Kügelchen in einem 1,5 ml Reaktionsgefäß wurden 10 µL DNA und 10 µL einer sterilen 4% PVA-Lösung (4 % Polyvinylalkohol in 200 mM Tris-HCl, pH 8,0) gegeben (gleiche Zusammensetzung wie Behandlung 3).
Behandlung 6: Zu etwa 55 Kügelchen in einem 1,5 ml Reaktionsgefäß wurden 10 µL DNA, 4 µL einer sterilen 4% PVA-Lösung (4% Polyvinylalkohol in 200 mM Tris-HCl, pH 8,0) und 6 µL Wasser gegeben.
Behandlung 7: Zu etwa 55 Kügelchen in einem 1,5 ml Reaktionsgefäß wurden 10 µL DNA, 2 µL einer sterilen 4% PVA-Lösung (4 % Polyvinylalkohol in 200 mM Tris-HCl, pH 8,0) und 8 µL Wasser zugegeben.

DNA und Kügelchen der 6 Reaktionsgefäße wurden bei Raumtemperatur luftgetrocknet.

Zwei Wochen später wurden Klonierungsreaktionen mit einem Kügelchen des Inserts und einem Kügelchen des Vektors, wie zuvor für die Behandlungen 1 bis 4 beschrieben, durchgeführt. Die Transformation wurde wie für das vorhergehende Experiment beschrieben durchgeführt, was die folgende Anzahl von Kolonien ergab:
Behandlung 5: 250 weiße Kolonien, geschätzt 37500 für die gesamte Transformation.
Behandlung 6: 181 weiße Kolonien, geschätzt 27150 für die gesamte Transformation.
Behandlung 7: 172 weiße Kolonien, geschätzt 25800 für die gesamte Transformation.

Diese Ergebnisse zeigen, dass die Kügelchen für mindestens 2 Wochen bei Raumtemperatur stabil sein können und nach wie vor für eine effiziente Klonierung geeignet sind.

## Patentansprüche

1. Verfahren zur Ein-Topf-Ein-Schritt-Assemblierung von zwei oder mehr DNA-Molekülen zu mindestens einem rekombinanten DNA-Molekül, wobei die zwei oder mehr zu assemblierenden DNA-Moleküle jeweils flankiert sind von Restriktionsendonuklease-Typ-IIs-Schnittstellen mit entgegengesetzter Orientierung und in einem Reaktionsgefäß mit einem geeigneten Reaktionsmedium zusammen gebracht werden, dadurch gekennzeichent, dass die zwei oder mehr zu assemblierenden DNA-Moleküle in trockener Form auf oder in mindestens einem Träger vorliegen, der so ausgestaltet und dimensioniert ist, dass er individuell als Ganzes und unverändert in einem Mikroreaktionsgefäß angeordnet werden kann und ein Filterpapierstück oder ein Zellulosepartikel ist oder umfasst.

2. Verfahren nach Anspruch 1, wobei die zwei oder mehr DNA-Moleküle jeweils auf oder in einem separaten Träger in getrockneter Form vorliegend in dem Reaktionsgefäß mit dem Reaktionsmedium zusammen gebracht werden.

3. Verfahren nach einem der vorhergehenden Ansprüche, wobei die zwei oder mehr DNA-Moleküle in einem Reaktionsmedium zusammen gebracht werden, das a) eine Restriktionsendonuklease vom Typ IIs und eine DNA-Ligase oder b) eine Exonuklease, eine DNA-Polymerase und eine DNA-Ligase enthält.

4. Träger für den Einsatz in einem Verfahren nach einem der Ansprüche 1 bis 3, umfassend mindestens ein darauf oder darin in trockener Form vorliegendes DNA-Molekül, das mit einem anderen DNA-Molekül assemblierbar ist und jeweils flankiert ist von Restriktionsendonuklease-Typ-IIs-Schnittstellen mit entgegengesetzter Orientierung, wobei der Träger mit dem DNA-Molekül so ausgestaltet und dimensioniert ist, dass er individuell als Ganzes und unverändert in einem Mikroreaktionsgefäß angeordnet werden kann, und wobei der Träger ein Filterpapierstück oder ein Zellulosepartikel mit dem darin enthaltenen DNA-Molekül ist oder umfasst.

5. Träger nach Anspruch 4, wobei der Träger das DNA-Molekül in einer Menge umfasst, die für eine Assemblierungsreaktion insbesondere nach dem "Golden-Gate"-Verfahren ausreicht.

6. Kit zur Durchführung des Verfahrens nach einem der Ansprüche 1 bis 3, umfassend mindestens einen Träger nach Anspruch 4 oder 5.

7. Kit nach Anspruch 6, umfassend zwei oder mehr zu assemblierende DNA-Moleküle, wobei jedes der zwei oder mehr DNA-Moleküle auf oder in einem separaten Träger in einer für die Assemblierung ausreichenden Menge vorliegt.

8. Kit nach einem der Ansprüche 6 oder 7, weiterhin umfassend a) eine Restriktionsendonuklease vom Typ IIs und eine DNA-Ligase oder b) eine Exonuklease, eine DNA-Polymerase und eine DNA-Ligase.

## Claims

1. A method for one-pot one-step assembly of two or more DNA molecules to form at least one recombinant DNA molecule, wherein the two or more DNA molecules to be assembled are respectively flanked by type IIs restriction endonuclease cleavage sites with opposite orientations and are brought together with a suitable reaction medium in a reaction vessel, **characterized in that** the two or more DNA molecules to be assembled are present in dry form on or in at least one substrate which is configured and dimensioned in a manner such that it can be disposed individually as a whole and unchanged in a micro-reaction vessel and is or comprises a piece of filter paper or a cellulose particle.

2. The method as claimed in claim 1, wherein the two or more DNA molecules are brought together with the reaction medium in the reaction vessel respectively on or in a separate substrate in dry form.

3. The method as claimed in one of the preceding claims, wherein the two or more DNA molecules are brought together in a reaction medium which contains a) a type IIs restriction endonuclease and a DNA ligase or b) an exonuclease, a DNA polymerase and a DNA ligase.

4. A substrate for use in a method according to one of claims 1 to 3, comprising at least one DNA molecule present thereon or therein in dry form, which can be assembled with another DNA molecule and is respectively flanked by type IIs restriction endonuclease cleavage sites with opposite orientations, wherein the substrate with the DNA molecule is configured and dimensioned in a manner such that it can be disposed individually as a whole and unchanged in a micro-reaction vessel, and wherein the substrate is or comprises a piece of filter paper or a cellulose particle with the DNA molecule contained therein.

5. The substrate as claimed in claim 4, wherein the substrate comprises the DNA molecule in a quantity which is sufficient for an assembly reaction, in particular in accordance with the "Golden Gate" method.

6. A kit for carrying out the method as claimed in one of claims 1 to 3, comprising at least one substrate as claimed in claim 4 or claim 5.

7. The kit as claimed in claim 6, comprising two or more DNA molecules to be assembled, wherein each of the two or more DNA molecules is present on or in a separate substrate in a quantity which is sufficient for the assembly.

8. The kit as claimed in claim 6 or claim 7, further comprising a) a type IIs restriction endonuclease and a DNA ligase or b) an exonuclease, a DNA polymerase and a DNA ligase.

## Revendications

1. Procédé visant à assembler, dans un récipient unique et en une seule étape, au moins deux molécules d'ADN de manière à obtenir au moins une molécule d'ADN recombinante, lesdites au moins deux molécules d'ADN à assembler étant encadrées par des sites de clivage d'endonucléase de restriction de type II à orientation opposée et étant réunies dans un récipient de réaction avec un milieu de réaction approprié, **caractérisé en ce que** lesdites au moins deux molécules d'ADN à assembler sont disposées sous une forme sèche sur ou dans au moins un support lequel est réalisé et dimensionné de manière à ce qu'il puisse être placé dans un récipient pour micro-réactions individuellement dans son entièreté et sans avoir subi de modifications et lequel correspond à un morceau de papier-filtre ou à une particule de cellulose ou comprend un tel / une telle.

2. Procédé selon la revendication 1, lesdites au moins deux molécules d'ADN étant réunies dans ledit récipient de réaction avec ledit milieu de réaction de manière à ce qu'elles soient chacune disposée sous une forme sèche sur ou dans un support séparé.

3. Procédé selon l'une des revendications précédentes, lesdites au moins deux molécules d'ADN étant réunies dans un milieu de réaction contenant a) une endonucléase de restriction de type II et une ligase d'ADN ou b) une exonucléase, une polymérase d'ADN et une ligase d'ADN.

4. Support destiné à être mis en œuvre dans un procédé selon l'une des revendications 1 à 3, comprenant au moins une molécule d'ADN qui est disposée sous une forme sèche sur ou dans celui-ci, qui peut être assemblée avec une autre molécule ADN, et qui est encadrée par des sites de clivage d'endonucléase de restriction de type II à orientation opposée, ledit support pourvu de ladite molécule d'ADN étant réalisé et dimensionné de manière à ce qu'il puisse être placé dans un récipient pour microréactions individuellement dans son entièreté et sans avoir subi de modifications, ledit support correspondant à un morceau de papier-filtre ou à une particule de cellulose ou comprenant un tel / une telle.

5. Support selon la revendication 4, ledit support comprenant ladite molécule d'ADN en une quantité qui est suffisante notamment pour une réaction d'assemblage selon la méthode dite "Golden Gate".

6. Kit destiné à mettre en œuvre le procédé selon l'une des revendications 1 à 3, comprenant au moins un support selon les revendications 4 ou 5.

7. Kit selon la revendication 6, comprenant au moins deux molécules d'ADN à assembler, chacune desdites au moins deux molécules d'ADN étant disposée, en une quantité qui est suffisante pour ledit assemblage, sur ou dans un support séparé.

8. Kit selon l'une des revendications 6 ou 7, comprenant en outre a) une endonucléase de restriction de type II et une ligase d'ADN ou b) une exonucléase, une polymérase d'ADN et une ligase d'ADN.


### SEQUENCE LISTING

<110> Leibniz-Institut für Pflanzenbiochemie (IPB)
<120> VERFAHREN, TRÄGER UND KIT ZUR EIN-TOPF-EIN-SCHRITT-ASSEMBLIERUNG VON
   DNA-MOLEKÜLEN
<130> PAT 1547 PCT
<150> DE10201610148.8
   <151> 2016-02-04
<160> 8
<170> BiSSAP 1.3.6
<210> 1
   <211> 15
   <212> DNA
   <213> Artificial Sequence
<220>
   <223> pICH50251 section
<400> 1
   ttaatgggag acctt 15
<210> 2
   <211> 15
   <212> DNA
   <213> Artificial Sequence
<220>
   <223> pICH50251 section
<400> 2
   aaggtctcag cttac 15
<210> 3
   <211> 15
   <212> DNA
   <213> Artificial Sequence
<220>
   <223> pAGM6752 section
<400> 3
   gtggtctcaa atggc 15
<210> 4
   <211> 15
   <212> DNA
   <213> Artificial Sequence
<220>
   <223> pAGM6752 section
<400> 4
   gagctttgag accac 15
<210> 5
   <211> 15
   <212> DNA
   <213> Artificial Sequence
<220>
   <223> pICH47732 section
<400> 5
   ccggagtgag accgc 15
<210> 6
   <211> 15
   <212> DNA
   <213> Artificial Sequence
<220>
   <223> pICH47732 section
<400> 6
   acggtctcac gctgc 15
<210> 7
   <211> 15
   <212> DNA
   <213> Artificial Sequence
<220>
   <223> pICH42301 section
<400> 7
   gtggtctcag gagag 15
<210> 8
   <211> 15
   <212> DNA
   <213> Artificial Sequence
<220>
   <223> pICH42301 section
<400> 8
   gacgcttgag accac 15